Europäisches Patentamt

European Patent Office

Office européen des brevets

(19)

(11) Veröffentlichungsnummer: **0 106 271**
**B1**

# EUROPÄISCHE PATENTSCHRIFT

(45) Veröffentlichungstag der Patentschrift:
15.01.86

(21) Anmeldenummer: 83109954.4

(22) Anmeldetag: 05.10.83

(51) Int. Cl.⁴: **B 01 J 31/40,** C 07 C 51/44,
C 07 C 53/122

(54) Verfahren zur Rückgewinnung und Wiederverwendung von Nickel, Kobalt oder Rhodium aus Gemischen, die Carbonsäure enthalten.

(30) Priorität: 19.10.82 DE 3238653

(43) Veröffentlichungstag der Anmeldung:
25.04.84 Patentblatt 84/17

(45) Bekanntmachung des Hinweises auf die Patenterteilung:
15.01.86 Patentblatt 86/3

(84) Benannte Vertragsstaaten:
BE DE FR GB IT NL

(56) Entgegenhaltungen:
EP-A-0 032 524
DE-B-1 014 093
US-A-2 609 337
US-A-3 497 460
US-A-3 641 137
US-A-4 060 557

(73) Patentinhaber: BASF Aktiengesellschaft, Carl-
Bosch- Strasse 38, D-6700 Ludwigshafen (DE)

(72) Erfinder: Broellos, Klaus, Dr., Floriansring 7,
D-6104 Seeheim- Jugenheim 1 (DE)
Erfinder: Lindner, Alfred, Dr., Ringstrasse 30,
D-6712 Bobenheim- Roxheim (DE)
Erfinder: Irnich, Rudolf, Dr., In den Hahndornen 2,
D-6719 Bobenheim (DE)
Erfinder: Hornberger, Paul, Dr., In den
Ziegelgaerten 12, D-6700 Ludwigshafen (DE)

## Beschreibung

Diese Erfindung betrifft ein Verfahren zur Rückgewinnung und Wiederverwendung von Nickel, Kobalt oder Rhodium aus Gemischen, die aliphatische Carbonsäuren enthalten.

Nickelkatalysatoren spielen bekanntlich in der Technik eine wichtige Rolle. Sie werden z.B. bei Hydrierungen, Hydrocarboxylierungen, Esterifizierungen und Crackreaktionen als feste Trägerkatalysatoren oder in gelöster flüssiger Form eingesetzt, wobei man das Nickel z.B. als Reinnickel, als Metallsalz oder metallorganische Komplexverbindung anwendet. Bei ihrem Einsatz erleiden diese Katalysatoren in Abhängigkeit der Betriebsdauer einen Aktivitätsverlust, so daß sie von Zeit zu Zeit regeneriert werden müssen.

Die Regenerierung von Nickelkatalysatoren, insbesondere ihre Abtrennung von organischen Verunreinigungen, bereitet erhebliche technische Schwierigkeiten. Beispielsweise verfährt man so, daß man die mit organischen Rückständen verunreinigten Nickelkatalysatoren verbrennt. Bei dieser aus den US-Patentschriften 1 306 871 und 4 120 698 bekannten Methode muß der aus Nickeloxid bestehende Rückstand anschließend reduziert werden. Nach dem in der US-A-4 120 698 beschriebenen Verfahren wird der den Katalysator enthaltende Rückstand auf Temperaturen von über 1000°C erhitzt. Die dabei erhaltene Schmelze wird zerkleinert und das Nickel magnetisch abgetrennt. Nicht minder umständlich ist das in der DE-A-30 00 040 beschriebene Verfahren, bei dem man das Nickel nach Lösen des verbrauchten Katalysators in Säuren elektrometallurgisch zurückgewinnt.

Diese Verfahren haben den weiteren Nachteil, daß das Nickel nicht in seiner katalytisch aktiven Form zurückgewonnen wird, sondern in diese noch übergeführt werden muß. Außerdem gehen verbrennbare Rückstände und damit Lösungsmittel und Wertprodukte verloren.

In der DE-C-10 14 093 wird ein Verfahren zur Abtrennung von Nickelcarbonylverbindungen aus Carbonylierungsprodukten beschrieben, bei dem man das heiße und unter Druck stehende Synthesegemisch entspannt, aus dem dabei erhaltenen flüssigen Produkt Nickelsalze und Nickel durch Filtration oder Sedimentation abtrennt, das flüssige Produkt dann destilliert und die im Sumpf zurückbleibenden restlichen Nickelsalze wieder in die Synthese zurückführt. Dieses Verfahren ist umständlich. Außerdem gelangen bei der Rückführung restlicher Nickelsalze Hochsiedesäuren in die Synthese, so daß Ausbeuteverluste eintreten und der Reaktor nach relativ kurzer Laufzeit entschlammt werden muß. Nach den Angaben der EP-A-00 32 524 arbeitet man den bei der Umsetzung von Olefinen mit Kohlenmonoxid und Alkoholen eingesetzten kobalthaltigen Katalysator, bei der man das Reaktionsgemisch oxidativ nachbehandelt, dadurch auf, indem man den Destillationssumpf mit einer 1 bis 4 Kohlenstoffatome enthaltenden Carbonsäure und mit Wasser behandelt, die wasserhaltige Phase abtrennt und das Kobalt in Form des entsprechenden carbonsäuren Salzes zurückgewinnt. Auch dieses Verfahren ist umständlich.

Es wurde nun gefunden, daß man Nickel, Cobalt oder Rhodium aus Gemischen, die aliphatische Carbonsäuren mit 3 bis 10 C-Atomen enthalten und die man durch Hydrocarboxylierung von Olefinen in Gegenwart von Nickel, Cobalt oder Rhodium enthaltenden Katalysatoren erhält, erheblich vorteilhafter zurückgewinnen und wiederverwenden kann, wenn man aus dem Gemisch in einen Dünnschicht erdampfer bei Temperaturen von 100 bis 200°C und einem Vakuum von 10 bis 50 mbar die Carbonsäuren weitgehend abdestilliert, zu dem die Metallsalze enthaltenden Sumpfprodukt reine Carbonsäure gibt und die dabei erhaltene Lösung der Metallsalze in der zurückgeführten Säure erneut für eine Hydrocarboxylierung heranzieht.

Die Ausgangsgemische für die Rückgewinnung von Nickel, Cobalt oder Rhodium nach dieser Erfindung sind Rückstände, die bei der an sich bekannten Hydrocarboxylierung von Olefinen in Gegenwart von Katalysatoren, die die genannten Metalle enthalten, erhalten werden. Sie bestehen aus metallhaltigen Gemischen aliphatischer Monocarbonsäuren mit 3 bis 10 C-Atomen. Diese hochsiedenden Gemische fallen z.B. bei der Propionsäuresynthese als Destillationsrückstand an. Sie enthalten als Nebenprodukte z.B. Essigsäure, Buttersäure, n-Valeriansäure, iso-Valeriansäure, Bernsteinsäure, n-Hexansäure und die Metalle sowie deren carbonsaure Salze, wie Nickelacetat, Nickelpropionat oder Nickelbutyrat. Gemische der genannten Art haben z.B. folgende Zusammensetzung:

Propionsäure 5 bis 75 Gew.%
Essigsäure 0 bis 1,5 Gew.%
Buttersäure 0 bis 1 Gew.%
Valeriansäure 10,0 bis 50 Gew.%
Methylbuttersäure 0,8 bis 4 Gew.%
Dimethylvateriansäure 0,5 bis 20 Gew.%
$C_7$- bis $C_{10}$-Säuren 5 bis 25 Gew.%
Nickel 0,1 bis 3 Gew.%

Die metallhaltigen Säuregemische werden erfindungsgemäß in einem zweckmäßigerweise senkrecht angeordneten Dünnschichtverdampfer, der vorzugsweise als Kolonne ausgestattet ist, unter fast vollständiger destillativer Entfernung der Carbonsäuren eingedampft. Bevorzugt sind Dünnschichtverdampfer deren innere Oberfläche beheizbar ist, z.B. durch eine Elektroheizung oder durch indirekte Beheizung mit einer Heizflüssigkeit. Zur besseren Verteilung des die Metalle enthaltenden Rückstandsstroms empfiehlt es sich, mechanisch bewegte Wischer zu verwenden, die die Heizfläche des Dünnschichtverdampfers teilweise oder ganz bestreichen. Geeignete Verdampfer sind beispielsweise als Sambay- oder Luwa-Dünnschichtverdampfer bekannt.

Der Dünnschichtverdampfer besteht aus zwei Temperaturzonen. Der untere Teil, der den Sumpf enthält, wird nicht beheizt. Er wird auf Raumtemperatur, d.h. Temperaturen bis etwa 35°C, zweckmäßig auf 20 bis 30°C gehalten. Die darüberliegende beheizte Zone, in die man das Ausgangsgemisch einleitet, wird bei Temperaturen von 100 bis 200°C und einem Vakuum von 10 bis 50 mbar so betrieben, daß aus den Ausgangsgemischen die Carbonsäuren weitgehend abdestilliert werden, wobei das Sumpfprodukt nahezu zur Trockene eingeengt wird. Im Sumpfprodukt liegt das Metall in Form von Salzen der hochsiedenden Carbonsäuren vor.

Zum Sumpfprodukt wird laufend reine Carbonsäure oder bei der Destillation aus dem Dünnschichtverdampfer abdestillierte Carbonsäure gegeben, wodurch man einen pumpfähigen Rückstand erhält, der die Metallsalze in gelöster Form enthält. Die so erhaltene Lösung der Metallsalze kann unmittelbar in dieser Form erneut als Katalysator für eine Hydrocarboxylierung zur Herstellung der gleichen Carbonsäure herangezogen werden. Die in der Lösung enthaltenen geringen Anteile an Ruß oder feinverteiltem Koks stören dabei nicht. Als Carbonsäure, die man in den Sumpf des Dünnschichtverdampfers zurückleitet, verwendet man vorzugsweise die reine Carbonsäure, die als Hauptprodukt der Carbonsäuresynthese anfällt. Man führt hierbei zweckmäßigerweise 10 bis 90, vorzugsweise 12 bis 50 Gew.% der abdestillierten Carbonsäure in den Sumpf zurück.

Nach dem erfindungsgemäßen Verfahren kann man die bei der Synthese von Carbonsäuren durch Hydrocarboxylierung benötigten Nickel-, Kobalt- oder Rhodium-Katalysatoren kontinuierlich rückgewinnen und regenerieren. Im Verbund mit der Carbonsäuresynthese verfährt man dabei z.B. so, daß man einen Teilstrom von etwa 1 bis 4 % der metallhaltigen Lösung, die man bei der Carbonsäuresynthese erhält, für die Behandlung nach dem Verfahren dieser Erfindung abzweigt. Man erzielt dabei den Vorteil einer besonders einfachen kontinuierlichen Rückgewinnung der Metalle und sichert dadurch einen störungsfreien Dauerbetrieb bei der Hydrocarboxylierung, der mit einer weitgehenden Minimierung des Verlustes an Wertprodukten aus dem organischen Rückstand verbunden ist.

Am Beispiel einer Hydrocarboxylierung von Ethylen in Gegenwart von Ni(CO)$_4$ als Katalysator, bei der man als Hauptprodukt Propionsäure erhält, soll das erfindungsgemäße Verfahren wie folgt erläutert werden (s. Figur):

Ein Teilstrom von 1 % der nickelhaltigen Zulauflösung zur Propionsäuresynthese, die die oben bezeichnete Zusammensetzung hat, wird in einem Dünnschichtverdampfer (2), der als Kolonne ausgebildet ist, eingeleitet (1). Der obere Teil der Kolonne ist beheizt, der untere Teil (3) ist nicht beheizt. Die Carbonsäuren werden im Vakuum bei 10 bis 50 mbar und im Siedebereich von 120 bis 200°C nahezu vollständig über den Kopf des Verdampfers abdestilliert (4). Im Sumpf der Kolonne (3) verbleibt das gesamte Nickel in Form von Salzen hochsiedender Säuren. Das Sumpfprodukt hat eine Temperatur von etwa 30°C. Die abdestillierten Carbonsäuren werden in eine Kolonne (5) geleitet (4), in der man reine Propionsäure von den höher siedenden Säuren abtrennt. Die höher siedenden Säuren werde als Rückstand abgetrennt (10) und gesondert aufgearbeitet oder verbrannt. Ein Teilstrom der über den Kopf der Kolonne erhaltenen reinen Propionsäure (6) wird über die Zuleitung (9) mit einer Temperatur von etwa 30°C in den Sumpf der Kolonne (3) geleitet. Man kann auch reine Propionsäure über die Leitung (8) zugeben. Die überschüssige reine Propionsäure (7) kann in die Hydrocarboxylierung zurückgegeben werden. Das flüssige Sumpfprodukt aus dem Dünnschichtverdampfer wird erneut für die Hydrocarboxylierung herangezogen (11).

**Beispiel 1**

a) Hydrocarboxylierung

Auf an sich übliche Weise wurden in einem Hochdruckautoklaven 2,5 Nm$^3$ Ethylen und 2,85 Nm$^3$ Kohlenmonoxid in flüssiger Phase einer 42 gew.%igen wäßrigen Propionsäurelöschung bei 240 bar und 280°C umgesetzt. Der wäßrigen Propionsäure wurde eine 2 bis 3 gew.%ige Nickelsalzlösung zugesetzt. Unter den Reaktionsbedingungen trat die Bildung von Nickelcarbonyl ein. Nach der Umsetzung wurde überschüssiges Wasser, der Propionsäureester und Propionsäure abdestilliert. Als Destillationsrückstand wurden 0,1 kg eines Nickel enthaltenden Säuregemisches erhalten.

b) Nickel-Rückgewinnung

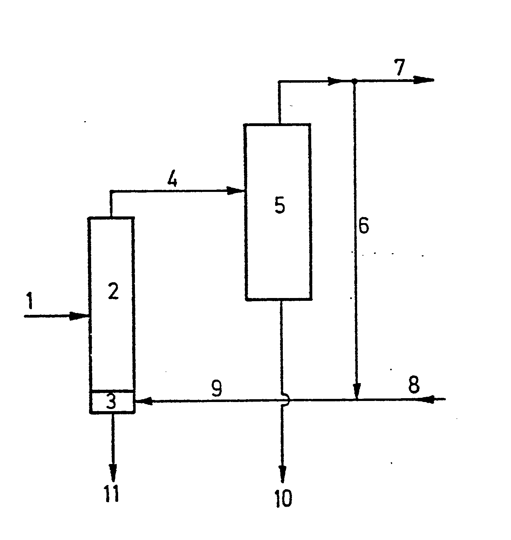

Das nach Absatz (a) erhaltene Säuregemisch hatte folgende Zusammensetzung: 50 bis 51 Gew.% Propionsäure, 0,5 bis 1 Gew.% aliphatische C$_5$- bis C$_6$-Carbonsäuren, 12 bis 13 Gew.% Dimethylvaleriansäure, 6,5 bis 7 Gew.% aliphatische C$_7$- bis C$_{10}$-Carbonsäuren und 0,15 Nickel. Nach dem in der Figur angegebenen Verfahrensschema wurden 600 g/h des genannten Säuregemisches (1) in den senkrecht angeordneten Dünnschichtverdampfer (2) eingebracht. Bei einer Sumpftemperatur von 30°C und einer Kopftemperatur von 100°C wurden 569 g/h Destillat (4) über Kopf des Dünnschichtverdampfers in eine Trennkolonne (5) geleitet. In der Kolonne wurde das Destillat des Dünnschichtverdampfers in 285 g/h 97 %ige Propionsäure und 285 g/h höher siedende Säuren getrennt. Ein Teilstrom der Propionsäure, nämlich 67 g/h, werden über Leitung (6) in den Sumpf des Verdampfers (3) zurückgeleitet. Am unteren Ende des Verdampfers werden als Rückstand 99,9 % des einges etzten Nickels erhalten. Die im Säuregemisch enthaltene Propionsäure wurde zu 99,7 % zurückgewonnen.

## Beispiel 2

Ein entsprechend Beispiel 1, Absatz (a) erhaltenes Säuregemisch hatte die folgende Zusammensetzung:

Propionsäure 9 Gew.%
Buttersäure 0,6 Gew.%
Valeriansäure 47 Gew.%
Methylbuttersäure 3 Gew.%
Dimethylvaleriansäure 17 Gew.%
n-Hexansäure-C$_{10}$-Säure 23 Gew.%
Nickel 0,4 Gew.%

Nach dem in der Figur angegebenen Verfahrensschema wurden 360 g/h dieses Gemisches entsprechend den Angaben von Beispiel 1, Absatz (6) behandelt. Dabei wurden 50 g/h eines Sumpfstromes (11) erhalten, der das vollständig abgetrennte Nickel (1,3 g/h) erhielt und erneut in der Synthese eingesetzt wurde.

Über Kopf des Dünnschichtverdampfers (2) wurden 350 g/h Destillat (als Strom 4) erhalten und in die Kolonne (5) geleitet. Es wurden 310 g/h höher siedende Säuren (10) und über Kopf der Kolonne (5) 40 g/h reine Propionsäure erhalten. Die reine Propionsäure wurden in den Sumpf des Dünnschichtverdampfers (3) zurückgeleitet. Das Nickel wurde zu 99,9 % und die Propionsäure zu 94,7 % zurückgewonnen.

## Patentansprüche

1. Verfahren zur Rückgewinnung und Wiederverwendung von Nickel, Cobalt oder Rhodium aus Gemischen, die aliphatische Carbonsäuren mit 3 bis 10 C-Atomen enthalten und die man durch Hydrocarboxylierung von Olefinen in Gegenwart von Nickel, Cobalt oder Rhodium enthaltenden Katalysatoren erhält, dadurch gekennzeichnet, daß man aus dem Gemisch in einem Dünnschichtverdampfer bei Temperaturen von 100 bis 200°C und einem Vakuum von 10 bis 50 mbar die Carbonsäuren weitgehend abdestilliert, zu dem die Metallsalze enthaltenden Sumpfprodukt reine Carbonsäure gibt und die dabei erhaltene Lösung der Metallsalze in der zurückgeführten Säure erneut für eine Hydrocarboxylierung heranzieht.

2. Verfahren nach Anspruch 1, dadurch gekennzeichnet, daß man Nickel aus Gemischen zurückgewinnt, die Propionsäure enthalten und die man durch Hydrocarboxylierung von Ethylen in Gegenwart von Nickel enthaltenden Katalysatoren erhält.

## Claims

1. A process for the recovery of nickel, cobalt or rhodium from mixtures which contain aliphatic carboxylic acids of 3 to 10 carbon atoms, and are obtained by hydrocarboxylating olefins in the presence of a nickel-, cobalt- or rhodium- containing catalyst, and the re-use thereof, wherein the carboxylic acids are substantially distilled off from the mixture in a thin-film evaporator at a temperature of from 100 to 200°C and in a vacuum of 10 to 50 millibars, pure carboxylic acid is added to the bottom product containing the metal salts, and the resulting solution of the metal salts in the recycled acid is used again for a hydrocarboxylation.

2. A process as claimed in claim 1, wherein nickel is recovered from mixtures which contain propionic acid and are obtained by hydrocarboxylating ethylene in the presence of a nickel containing catalyst.

## Revendications

1. Procédé pour la récupération et le recyclage du nickel, du cobalt ou du rhodium de mélanges contenant des acides carboxyliques aliphatiques en C$_3$ à C$_{10}$ et produits lors d'une hydrocarboxylation d'oléfines en présence de catalyseurs au nickel, au cobalt ou au rhodium, caractérisé en ce que l'on élimine les acides carboxyliques dans une large mesure par une distillation du mélange dans un évaporateur en couche mince, sous un vide de 10 à 50 millibars et à des températures de 100 à 200°C, on ajoute au produit résiduel contenant les sels de métaux de l'acide carboxylique pur et on utilise la solution des sels de métaux dans l'acide recyclé pour une nouvelle hydrocarboxylation.

2. Procédé suivant la revendication 1, caractérisé en ce qu'il est appliqué à la récupération du nickel de mélanges contenant de l'acide propionique et produits lors de l'hydrocarboxylation de l'éthylène en présence d'un catalyseur au nickel.